# EUROPEAN PATENT SPECIFICATION

(11) **EP 3 474 732 B1**
(45) Date of publication and mention of the grant of the patent: **06.12.2023**
(21) Application number: 17746368.4
(22) Date of filing: 22.06.2017
(51) Int. Cl.: A61B 5/00, A61B 5/024, A61B 5/0245, A61B 5/08, A61B 5/11, A61B 5/113

(54) **DEVICE FOR MONITORING PATIENT'S VITAL FUNCTIONS COMPRISING A PIEZOELECTRIC TRANSDUCER AND A MEASURING CAPACITOR**
VORRICHTUNG ZUR ÜBERWACHUNG DER LEBENSFUNKTIONEN EINES PATIENTEN, BESTEHEND AUS EINEM PIEZOELEKTRISCHEN WANDLER UND EINEM MESSKONDENSATOR
DISPOSITIF DE SURVEILLANCE DES FONCTIONS VITALES D'UN PATIENT COMPRENANT UN TRANSDUCTEUR PIEZOELECTRIQUE ET UN CONDENSATEUR DE MESURE

(30) Priority: 22.06.2016 CZ 20160366
(43) Date of publication of application: 01.05.2019
(73) Proprietor: Linet Spol. S.R.O., 27401 Slany (CZ)
(72) Inventor: KOLÁR , Vladimir, Slaný 27401 (CZ)
(86) International application number: PCT/CZ2017/000041
(87) International publication number: WO 2017/220055

(56) References cited:
- WO-A1-2011/018706
- WO-A1-2015/051770
- US-A1- 2015 204 744
- US-A1- 2015 282 753

## Description

### Technical field

The invention relates to a device for monitoring patient's vital functions such as respiratory and heart rate and changes or arrhythmia thereof.

### Background of the Invention

Various devices for monitoring various vital functions are known in the art. These monitoring devices are used especially in hospitals, mostly in intensive care units, but also in aftercare departments or in nursing homes. Devices are known where there is no need for a direct connection with the patient, which facilitates patient care, as there is no need for patient's cooperation. The direct contact with the patient means various types of for example adhesive sensors or sensors that are inserted into the patient's body.

Majority of the known devices consist of a pad which incorporates one or more sensors of different types. These sensors can detect a change in force applied to the pad, for example by means of various force sensors with offset measurement, strain gauges or sensors employing piezoelectric effect. Furthermore, the sensors can also detect vibrations of the bed-deck caused by the patient, for example, by using various MEMS (micro-electromechanical sensor) sensors. The pad is placed under the area where the patient is located, usually under the mattress.

Device employing appropriately configured strain gauges is described in patent US7699784. The disadvantage of this solution is that there is often an unwanted reaction to the surrounding forces, due to the configuration of the strain gauges so that the patient's breathing and heartbeat can be recorded.

Devices employing the principle of direct piezoelectric effect are utilized because of their low price and simplicity in measuring high-frequency vital functions, such as heartbeat. An example of such device is disclosed in patent US6984207. However, these devices are not suitable for measuring low-frequency vital functions, such as breathing.

For measuring low-frequency vital functions, sensors for measuring offset are used, for example, capacity sensors utilizing capacity changes due to change in the size of the air gap between the electrodes, as disclosed, for example, in patent application WO2006131855.

The device can also be as disclosed in patent application WO2010080794 where the pad is filled with fluid, and the pressure sensor senses changes in the pressure caused by breathing and patient's pulse. The problem with this solution is in the production complexity of the special pad filled with liquid.

The device can also employ the video signal evaluation principle as disclosed in application WO2013027027, where it is possible to evaluate some vital functions on the basis of the light intensity ratio of two different wavelengths reflected from the patient's skin. However, this principle is inaccurate and very difficult to perform in poor lighting conditions.

The device can also be formed by mattresses into which the sensor is inserted, as disclosed in patent US7652581. However, this method is disadvantageous due to the high price of the mattress adapted for this purpose.

Indirect measurement of the patient's vital functions raises sensor accuracy requirements so that they are able to respond also to minor changes caused by breathing or patient's heartbeat and at the same time so that they are not disturbed by undesirable external forces. Meeting these requirements can be achieved by using a combination of sensors, which increases the cost of this device. This problem is partially solved by patent application CZ2013-781 (published in PCT phase as WO 2015/051770 A1), which utilizes a sensor consisting of three conductive electrodes, where one electrode is a piezoelectric sensor and a second electrode together with a third electrode forms a capacity sensor. However, this solution still has high production costs, mechanical complexity and insufficient precision. The disadvantage of this device is also that the piezoelectric sensor is made of a mixture of PolyVinilDene Fluoride and Polyethylene terephthalate material, such sensor has a number of drawbacks in the said application of measuring patient's vital functions. Above all, this material is insufficiently stiff, resulting in the need to regulate the forces applied on the sensor with a rigid, stiffer cover. Furthermore, this sensor needs to be supported from one side by a spring to avoid excessive vibration. The piezoelectric sensor also has a disadvantage in the electromechanical properties of the used material, with an elongation of 1% there is a charge in the order of Coulomb units which does not allow sufficient accuracy of either measuring vital functions, such as heartbeat and breathing, or detecting weaker signals such as peristaltic body manifestations.

### Summary of the Invention

The abovementioned drawbacks are removed by a device for monitoring patient's vital functions as defined in the independent claim 1. The device comprises a computing unit and a piezoelectric transducer. The advantage lies in the fact that it further comprises a measuring electrode coupled to a board forming a measuring capacitor together with the piezoelectric transducer. The piezoelectric transducer and the measuring capacitor are electrically connected to the computing unit. The piezoelectric transducer is deformable and is propped against the board on at least two places so that a flexible part of the piezoelectric transducer is located between these places. The place for the bent portion of the piezoelectric transducer is achieved, for example, by the saucer-type shape of the piezoelectric transducer or by placing the piezoelectric transducer on the elevated place of the board or through the hole in the board. This advantage is utilized for mechanical simplification of the design and simultaneously for reduction of the failure rate.

In the device according to the invention, the piezoelectric transducer comprises a first electrode and a second electrode and a piezoelectric element formed by piezoelectric material located therebetween. The first electrode and the second electrode are electrically connected with the computing unit. In a preferred embodiment, the piezoelectric element is formed by piezoceramics.

Preferably it is utilized that the second electrode has a circular shape.

In the device according to the invention, the measuring electrode forms together with the first electrode or the second electrode the measuring capacitor.

Preferably it is utilized that it further comprises a printed circuit board. The computing unit, the piezoelectric transducer and the measuring capacitor are located on the printed circuit board.

Preferably it is utilized that the second electrode or the first electrode is attached in three places of the perimeter of the area adjoining the printed circuit board attached so that each attachment allows the deformation of the piezoelectric transducer.

Preferably it is utilized that it further includes a comparator capacitor. The comparator capacitor is electrically connected with the computing unit.

Preferably it is utilized that the comparator capacitor comprises a first comparator electrode and a second comparator electrode. The first comparator electrode and the second comparator electrode are electrically connected with the computing unit. Preferably it is utilized that it further includes a sampling capacitor. The sampling capacitor is electrically connected with the computing unit.

Preferably it is utilized that it further includes a shielding electrode of the measuring capacitor. The shielding electrode of the measuring capacitor is electrically connected with the computing unit. The shielding electrode of the measuring capacitor has the same electrical potential as the measuring electrode. In a preferred embodiment, it also includes a shielding electrode of the comparator capacitor. The shielding electrode of the measuring capacitor is electrically connected to the computing unit. The shielding electrode of the comparator capacitor has the same electrical potential as the first comparator electrode or the second comparator electrode.

Preferably it is utilized that it further comprises the printed circuit board. The shielding electrode of the measuring capacitor, the shielding electrode of the comparator capacitor, the comparator capacitor and the sampling capacitor are located on the printed circuit board.

It is preferably utilized that the shielding electrode of the measuring capacitor is located opposite of the measuring electrode and the shielding electrode of the comparator capacitor is located opposite of the first comparator electrode or the second comparator electrode.

### Exemplary Embodiment of the Invention

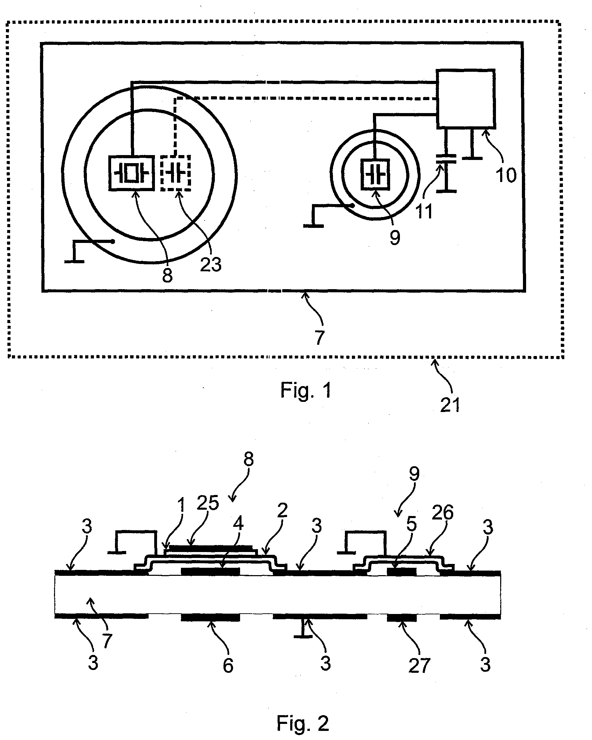

An exemplary embodiment of the invention is a device **21** for monitoring patient's vital functions according to fig. 1, fig. 2 and fig. 3 comprising a piezoelectric transducer **8**, a comparator capacitor **9**, a printed circuit board **7**, a plating **3** and a computing unit **10**. The plating **3** may be, for example, of copper. The computing unit **10**, according to the preferred embodiment, consists of a single processor, but may also consist of other computing parts communicating with each other by wire or wirelessly.

The piezoelectric transducer **8** utilized here for the purpose of measuring vital functions is a component for generating sound, namely an electro-acoustic transducer used in watches, as a siren or as a buzzer. The piezoelectric transducer has suitable mechanical-deformative properties for measuring vital functions, which means that springs and additional members transferring their deformations caused by movements associated with patient's vital functions to the measuring element are not necessary in comparison with prior art devices, thus achieving low production costs, higher measuring accuracy, and simplicity of the entire device **21**. A semiconductor component and a circuit utilized for measuring capacity in touchscreen displays (charge transfer technology/method) are utilized in measuring capacity with this component and another measuring (static) electrode **4**. A specific exemplary embodiment is described below.

In the exemplary embodiment, the device **21** is on the side of the piezoelectric transducer **8** covered by, for example, a plastic diaphragm which protects the device **21** against water and dust and at the same time also removes the mechanical resonance oscillation of the device **21.** The device **21** is on the side opposite of the piezoelectric transducer **8** covered by for example plastic foil which ensures the minimum height of the entire device **21**. The plastic film may be replaced by any flexible or movable cover which ensures the transfer of forces to the piezoelectric transducer **8** without its stiffness significantly affecting the resulting force being transmitted to the piezoelectric transducer **8**. The device **21** for monitoring patient's vital functions may be adapted for insertion into the pad. The pad can be stored, for example, between the mattress and the bed frame, in the mattress or between the mattress and the patient's body. The device may also be adapted for a direct placement on the bed frame and for detachable locking, for example, with a snap fastener.

The piezoelectric transducer **8** consists of the piezoelectric element **1** from piezoceramics, the first electrode **25** and the second electrode **2**. Piezoceramics are for example piezoceramic materials based on lead zirconate titanate [Pb[ZrₓTi₁₋ₓ]O₃ with 0 ≤ x ≤ 1] or sodium bismuth titanate [NaBi(TiO₃)₂] or other piezoceramic material. By using such material, the required electromechanical properties are achieved, namely generation of charge in range of 130-930 pC/N. The piezoelectric element **1** is located between the first electrode **25**, for example, from silver, optionally from alloys with similar electrical properties, and the second electrode **2**. The piezoelectric transducer **8** may have different shapes, such as a circle, a triangle, square, or other shapes. A preferred shape is the circular shape, which ensures the most uniform decomposition of forces. The shape of the piezoelectric transducer **8** is usually a circle delimited in its height dimension by two parallel planes. Preferably, form modification of the piezoelectric transducer **8** can be utilized, when its center is pressed in the direction of axis perpendicular to one of the delimiting planes and a board-like shape emerges, where the center is located in one plane and in parallel plane lie the borders of the piezoelectric transducer **8**. The board-like shape is best illustrated by the shape of the second electrode **2** (or the entire piezoelectric transducer **8**) in Figure 2. The second electrode **2** may be made of brass, aluminum, copper or another metallic material. The locations of the electrodes are interchangeable, it is essential that the piezoelectric element **1** is positioned in the middle between them. The second electrode **2** may have a circular or optionally board-like shape. Alternatively, the second electrode may have the shape of ellipse, polygon, most frequently of rectangle, square, or another shape. Shape of the second electrode **2** is preferably derived from the shape of the piezoelectric transducer **8**. The second electrode **2** is flat and its surface is in a rest position approximately parallel to the piezoelectric element **1**. The second electrode **2** is located on the plating **3** for electrical connection with the ground. This creates a firm connection with the printed circuit board **7**. The second electrode **2** is connected at least in two places to cause the required deformation of the piezoelectric transducer **8**. These two places may be located, for example, opposite of each other on the opposite sides of the length of the piezoelectric transducer **8**. In an alternative embodiment, the attachment can be provided so that that the first electrode **25** is located on the plating **3** and thereby the piezoelectric transducer **8** is connected with the printed circuit board **7**. In a preferred embodiment, the second electrode **2** is connected in at least three places adjoining the printed circuit board **7** to provide greater stability and better course of deformation of the piezoelectric transducer **8**. Preferably, these places are in the piezoelectric transducer with circular shape positioned so that they create a triangle when connected. Alternatively, the piezoelectric transducer **8** may be connected to any fixed board in this way, but by connecting to the printed circuit board **7**, the dimensions of the entire device **21** are minimized. The measuring capacitor **23** consists of the second electrode **2** and the measuring electrode **4.** The measuring electrode **4** is connected with the printed circuit board **7** and forms electrode with higher electrical potential. The dielectric of the measuring capacitor **23** is formed by an air gap. On the opposite side of the printed circuit board **7** opposite of the measuring electrode **4** there is the shielding electrode **6** of the measuring capacitor **23**. The shielding electrode **6** of the measuring capacitor **23** has the same electrical potential as the measuring electrode **4** and together they can form a shielding capacitor **24** which provides resistance to external influences for example by the approximation of metal material. In some cases, two or more piezoelectric transducers **8** may be located on one circuit board **7**.

The comparator capacitor **9** consists of the first comparator electrode **26**, the first comparator electrode **26** can be made, for example, of brass, aluminum, copper. The first comparator electrode **26** is located on the plating **3** for electrical connection with the ground. The comparator capacitor **9** further consists of a second comparator electrode **5**. The second comparator electrode **5** is located on the printed circuit board **7** and forms an electrode with higher electrical potential. The dielectric of the comparator capacitor **9** is formed by an air gap. On the opposite side of the printed circuit board **7** opposite of the second comparator electrode **5** is located the shielding electrode **27** of the comparator capacitor **9**. The shielding electrode **27** of the comparator capacitor **9** has the same electrical potential as the comparator electrode **5** and together they can form a shielding capacitor **24** which provides resistance to external influences for example by the approximation of metal material.

The computing unit **10** combines piezoelectric voltage measurement and capacity measurement functions, for example, by means of charge transfer technology. Piezoelectric element **1** is connected to the computing unit **10** through a charge amplifier. Furthermore, the measuring capacitor **23**, the comparator capacitor **9**, the shielding capacitor **24** and the sampling capacitor **11** are connected to the computing unit **10**. The use of the measuring capacitor **23** and the comparator capacitor **9** causes resistance to changes in the measuring conditions such as temperature or humidity.

Furthermore, a method for monitoring patient's vital functions according to the above-mentioned exemplary embodiment is described. Heartbeat, breath and other vital functions of the patient, for example peristalsis, generate forces transmitted to the device **21** for monitoring patient's vital functions. With respect to the construction of the device **21**, it is ensured that the vital functions of the patient are measurable without the need for permanent connection of the device **21** with the patient's body, for example by means of gluing or implanting. The device **21** is capable of measuring patent's vital functions in contact with the skin of the patient, but also in contact with patient's clothing, mediated through the mattress on which the patient is placed, or through the pad in which the device **21** for monitoring vital functions may be located. The measuring capacitor **23** and the comparator capacitor **9** are used to measure slowly changing forces generated by, for example, breathing. During breathing, due to the applied forces, deflection of the central portion of the second electrode **2** (and therefore to the entire piezoelectric transducer **8**) occurs, and thus also change in the air gap between the second electrode **2** and the measuring electrode **4**. Due to the construction where the piezoelectric transducer **8** is attached to the board by one of the electrodes, a non-mediated deflection of the piezoelectric transducer **8** without the need for additional force transmitting components is enabled. The size of the air gap between the first comparator electrode **26** and the second comparator electrode **5** is independent of the action of the forces. The change in the capacity of the measuring capacitor **23** is dependent both on the varying air gap size and on the change of permittivity of the air gap. The change in the capacity of the comparator capacitor **9** is dependent only on change in permittivity of the air gap. The capacity of the measuring capacitor **23** and the capacity of the comparator capacitor **9** ratio will remove the dependence of the capacitance change on dielectric permittivity and thereby the independence of changing the measuring conditions.

Capacity changes are evaluated for example by charge transfer technology. The charge transfer technology operates on the principle of charging the capacitor and subsequent transfer of the accumulated charge into the sampling capacitor **11**, wherein the number of accumulated charge transfers into the sampling capacitor is counted, until the voltage at the sampling capacitor **11** reaches the same value as the stable reference voltage. It is clear to a person skilled in the art that other methods of measuring capacity can be utilized, for example, the resonance method. In order to measure fast-changing forces caused for example by pulse, a direct piezoelectric effect of the piezoelectric material is used, where by deformation of the piezoelectric material due to external forces, a charge which is through the charge amplifier transferred to the computing unit **10** is generated where the voltage is evaluated. The output data are then transmitted via the data wire **22** to the control unit **20**. The computing unit **10** can be provided as a microprocessor, its location on the printed circuit board **7** ensures a protection of the signal because the distance of the transmitted non-digital signal is very small.

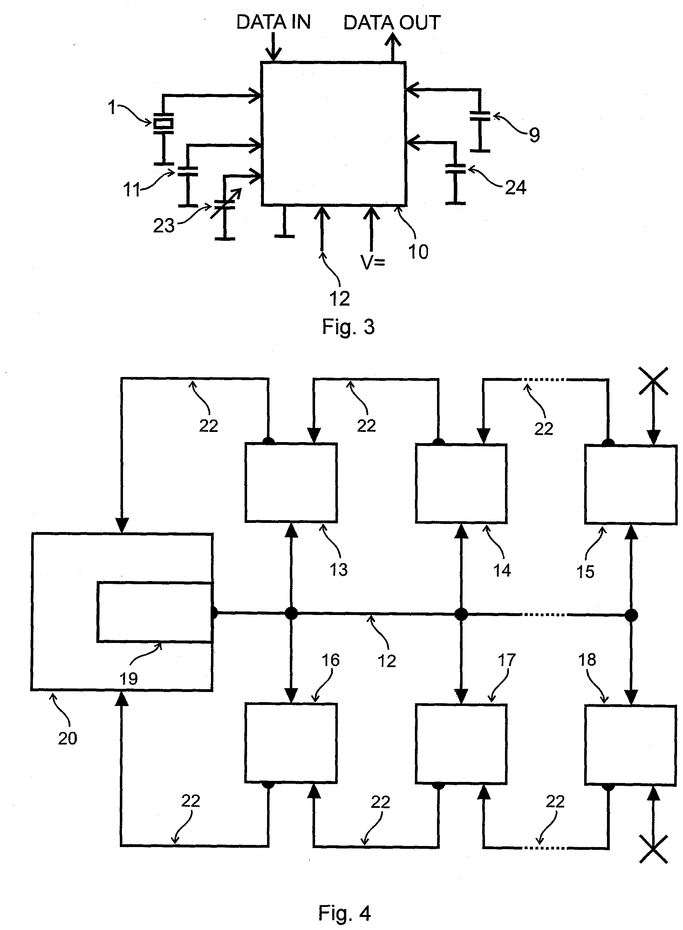

Further, a method for communication of the measuring elements A₁ to A_{N} **13**, **14**, **15** which may be devices for monitoring vital functions of a patient **21** or sensors of other type according to Fig. 4 is described. The pad contains a plurality of measuring elements A₁ to A_{N} **13**, **14**, **15** connected by at least one trigger wire **12** with the control unit **20**. The measurement elements A₂ through A_{N-1} are connected by the data wires **22** with the adjacent measuring elements and the measuring elements A₁ and A_{N} **13**,**15** are connected by the data wire **22** with the adjacent element. The measuring element A₁ **13** is connected by the data wire also with the control unit **20**. By connecting the measuring elements A₁ to A_{N} **13**, **14**, **15** by the data wires **22**, a serial data connection of these measuring elements A₁ to A_{N} **13**, **14**, **15**, is established, resulting in a reduction in the required amount of cableway. The trigger wire **12** is connected to the trigger **19**, task of which is to transmit the signal for initiation of the measurement and for transmission of the measured information. Upon receipt of the signal from the trigger **19**, the data message from each measuring element A_{K} is sent through the data wire **22** to the measuring element A_{K-1} which subsequently sends it through the data wire to the measuring element A_{K-2}, this way the data message is sent through the data wires **22** to the measuring element A₁ **13** from where it is sent through the data wire **22** to the control unit **20**, the transfer process is repeated until the data message from the A_{N} **15** is received in the control unit **20**. K takes values from interval 1 to N. The data messages are sent from all measuring elements A₁ to A_{N} **13**, **14**, **15** simultaneously, so that the data message from the measuring element A₁ **13** arrives first, then the data message from the measuring element A₂ **14** arrives and finally the data message from the measuring element A_{N} **15** arrives. The method described above describes only one branch of the measuring element A₁ **13**, the measuring element A₂ **14** to the measuring element A_{N} **15**, there can be more of these branches connected to the control unit **20** as, for example, in Figure 4, branch B, with measuring element B₁ **16**, measuring element B₂ **17** to measuring element B_{N} **18.**

### List of Reference Signs

- 1 -: piezoelectric element
- 2 -: second electrode
- 3 -: plating
- 4 -: measuring electrode
- 5 -: second comparator electrode
- 6 -: shielding electrode of measuring capacitor
- 7 -: printed circuit board)
- 8 -: piezoelectric transducer
- 9 -: comparator capacitor
- 10 -: computing unit
- 11 -: sampling capacitor
- 12 -: trigger wire
- 13 -: measuring element A₁
- 14 -: measuring element A₂
- 15 -: measuring element A_{N}
- 16 -: measuring element B₁
- 17 -: measuring element B₂
- 18 -: measuring element B_{N}
- 19 -: trigger
- 20 -: control unit
- 21 -: device for monitoring patient's vital functions
- 22 -: data wire
- 23 -: measuring capacitor
- 24 -: shielding capacitor
- 25 -: first electrode
- 26 -: first comparator electrode
- 27 -: shielding electrode of comparator capacitor

## Claims

1. Device for monitoring patient's vital functions comprising a computing unit, a measuring electrode (4), a piezoelectric transducer (8), wherein the piezoelectric transducer (8) comprises a first electrode (25) and a second electrode (2) and a piezoelectric element (1) formed by piezoelectric material, wherein the piezoelectric element (1) is positioned between the first electrode (25) and the second electrode (2), wherein the measuring electrode (4) forms, together with the first electrode (25) or the second electrode (2) of the piezoelectric transducer (8), a measuring capacitor (23), wherein the piezoelectric transducer (8) and the measuring capacitor (23) are electrically connected with the computing unit (10), wherein the measuring electrode (4) is connected to a board, wherein the piezoelectric transducer (8) is propped against the board, on which the measuring electrode (4) is located, in at least two positions, wherein between the at least two positions is located a flexible portion of the piezoelectric transducer (8) and wherein the computing unit (10) is configured to combine piezoelectric voltage measurement and capacity measurement function

2. Device for monitoring patient's vital functions according to claim 1 **characterized in that** the piezoelectric element (1) is formed by piezoceramics.

3. Device for monitoring patient's vital functions according to any of the preceding claims **characterized in that** the board is a printed circuit board (7) on which the computing unit (10), the piezoelectric transducer (8) and the measuring capacitor (23) are located.

4. Device for monitoring patient's vital functions according to claim 3 **characterized in that** the second electrode (2) or the first electrode (25) is attached in three places of the perimeter of the area of the adjoining printed circuit board (7) so that each attachment enables deformation of the piezoelectric transducer (8).

5. Device for monitoring patient's vital functions according to any of the preceding claims **characterized in that** it further comprises a comparator capacitor (9) which is electrically connected with the computing unit (10).

6. Device for monitoring patient's vital functions according to claim 5 **characterized in that** the comparator capacitor (9) comprises a first comparator electrode (26) and a second comparator electrode (5), wherein the first comparator electrode (26) and the second comparator electrode (5) are electrically connected with the computing unit (10).

7. Device for monitoring patient's vital functions according to any of the preceding claims **characterized in that** it further comprises a sampling capacitor (11) which is electrically connected with the computing unit (10).

8. Device for monitoring patient's vital functions according to claim 6 **characterized in that** it further comprises a shielding electrode (6) of the measuring capacitor (23) electrically connected with the computing unit (10) and having the same electrical potential as the measuring electrode (4) and/or a shielding electrode (27) of the comparator capacitor (9) electrically connected with the computing unit (10) which has the same electrical potential as the first comparator electrode (26) or the second comparator electrode (5).

9. Device for monitoring patient's vital functions according to claim 8 **characterized in that** any element chosen from the group of the shielding electrode (6) of the measuring capacitor (23), shielding electrode (27) of the comparator capacitor (9), the comparator capacitor (9) and the sampling capacitor (11) is located on the board (7).

10. Device for monitoring patient's vital functions according to any of claims 8 to 9 **characterized in that** the shielding electrode (6) of the measuring capacitor (23) is located opposite the measuring electrode (4) and the shielding electrode (27) of the comparator capacitor (27) is located opposite the first comparator electrode (26) or the second comparator electrode (5).

## Patentansprüche

1. Eine Einrichtung zur Überwachung der Vitalparameter (Lebensfunktionen) des Patienten, bestehend aus einen Computereinheit (10), einen Messelektrode (4), einen piezoelektrischen Wandler (8), wobei der piezoelektrische Wandler (8) ferner eine erste Elektrode (25) und eine zweite Elektrode (2) sowie ein piezoelektrische Element (1), bestehend aus piezoelektrischem Material, umfasst, wobei sich das piezoelektrische Element (1) zwischen der ersten Elektrode (25) und der zweiten Elektrode (2) befindet, wobei die Messelektrode (4) gemeinsam mit der ersten Elektrode (25) oder der zweiten Elektrode (2) des piezoelektrischen Wandlers (8) einen Messkondensator (23) bildet, wobei der piezoelektrische Wandler (8) und der Messkondensator (23) elektrisch mit der Computereinheit (10) verbunden sind, wobei die Messelektrode (4) mit einer Platte verbunden ist, **die dadurch gekennzeichnet ist, dass** der piezoelektrische Wandler (8) auf die Platte, auf der sich die Messelektrode (4) befindet, an mindestens zwei Punkten gestützt ist, wobei zwischen diesen mindestens zwei Punkten der flexible Teil des piezoelektrischen Wandlers (8) positioniert ist, indem die Computereinheit (10) die Funktionen der Messung der piezoelektrischen Spannung und der Messung der Kapazität kombiniert.

2. Die Einrichtung zur Überwachung der Lebensfunktionen (Vitalparameter) des Patienten gemäß dem Patentanspruch 1, **welches dadurch gekennzeichnet ist, dass** das piezoelektrische Element (1) aus Piezokeramik besteht.

3. Die Einrichtung zur Überwachung der Lebensfunktionen (Vitalparameter) des Patienten gemäß einem jedweden der vorherigen Patentansprüche, welches **dadurch gekennzeichnet ist, dass** die Platte eine Leiterplatte (7) ist, auf der die Computereinheit (10), der piezoelektrische Wandler (8) und der Messkondensator (23) positioniert sind.

4. Die Einrichtung zur Überwachung der Lebensfunktionen (Vitalparameter) des Patienten gemäß dem Patentanspruch 3, welches **dadurch gekennzeichnet ist, dass** die zweite Elektrode (2) oder die erste Elektrode (25) an drei Stellen des Umfangs der die Leiterplatte (7) berührenden Fläche so befestigt ist, dass jede Befestigung die Verformung des piezoelektrischen Wandlers (8) ermöglicht.

5. Die Einrichtung zur Überwachung der Lebensfunktionen (Vitalparameter) des Patienten gemäß einem jedweden der vorherigen Patentansprüche, welches **dadurch gekennzeichnet ist, dass** es ferner einen Referenzkondensator (9) umfasst, der elektrisch mit der Computereinheit (10) verbunden ist.

6. Die Einrichtung zur Überwachung der Lebensfunktionen (Vitalparameter) des Patienten gemäß dem Patentanspruch 5, **welches dadurch gekennzeichnet ist, dass** der Referenzkondensator (9) eine erste Referenzelektrode (26) und eine zweite Referenzelektrode (5) umfasst, wobei die erste Referenzelektrode (26) und die zweite Referenzelektrode (5) elektrisch mit der Computereinheit (10) verbunden sind.

7. Die Einrichtung zur Überwachung der Lebensfunktionen (Vitalparameter) des Patienten gemäß einem jedweden der vorherigen Patentansprüche, **welches dadurch gekennzeichnet ist, dass** es ferner einen Abtastkondensator (11) umfasst, der elektrisch mit der Computereinheit (10) verbunden ist.

8. Die Einrichtung zur Überwachung der Lebensfunktionen (Vitalparameter) des Patienten gemäß dem Patentanspruch 6, **welches dadurch gekennzeichnet ist, dass** es ferner eine Abschirmelektrode (6) des Messkondensators (23) umfasst, die mit der Computereinheit (10) elektrisch gekoppelt ist und das gleiche elektrische Potential wie die Messelektrode (4) hat, und/oder eine Abschirmelektrode (27) des Referenzkondensators (9) umfasst, die mit der Computereinheit (10) elektrisch verbunden ist und das gleiche elektrische Potential wie die erste Referenzelektrode (26) oder die zweite Referenzelektrode (5) aufweist.

9. Die Einrichtung zur Überwachung der Lebensfunktionen (Vitalparameter) des Patienten gemäß dem Patentanspruch 8, **welches dadurch gekennzeichnet ist, dass** auf der Leiterplatte (7) ferner ein jedwedes Element aus der Gruppe positioniert ist, die aus der Abschirmelektrode (6) des Messkondensators (23), der Abschirmelektrode (27) des Referenzkondensators (9), dem Referenzkondensator (9) und dem Abtastkondensator (11) besteht.

10. Die Einrichtung zur Überwachung der Lebensfunktionen (Vitalparameter) des Patienten gemäß einem jedweden der Patentanspruch 8 bis 9, **welches dadurch gekennzeichnet ist, dass** die Abschirmelektrode (6) des Messkondensators (23) gegenüber der Messelektrode (4) und die Abschirmelektrode (27) des Referenzkondensators (27) gegenüber der ersten Referenzelektrode (26) oder der zweiten Referenzelektrode (5) positioniert ist.

## Revendications

1. Dispositif de surveillance des fonctions vitales d'un patient comprenant une unité de calcul (10), une électrode de mesure (4) et un transducteur piézoélectrique (8), le transducteur piézoélectrique (8) comprenant en outre une première électrode (25) et une seconde électrode (2) et un élément piézoélectrique (1) constitué d'un matériau piézoélectrique, l'élément piézoélectrique (1) étant situé entre la première électrode (25) et la deuxième électrode (2), tandis que l'électrode de mesure (4) ainsi que la première électrode (25) ou la deuxième électrode (2) du transducteur piézoélectrique (8) forment un condensateur de mesure (23), le transducteur piézoélectrique (8) et le condensateur de mesure (23) étant connectés électriquement à l'unité de calcul (10) tandis que l'électrode de mesure (4) est reliée à une plaque, **caractérisé en ce que** le transducteur piézoélectrique (8) repose contre la plaque sur laquelle l'électrode de mesure (4) est placée en au moins deux points, sachant que la partie flexible du transducteur piézoélectrique (8) est placée entre ces au moins deux points, et que l'unité de calcul (10) combine les fonctions de mesure de tension piézoélectrique et de mesure de capacité.

2. Dispositif de surveillance des fonctions vitales du patient selon la revendication 1, **caractérisé en ce que** l'élément piézoélectrique (1) est en céramique piézoélectrique.

3. Dispositif de surveillance des fonctions vitales du patient selon l'une quelconque des revendications précédentes, **caractérisé en ce que** la plaque est une carte de circuits imprimés (7) sur laquelle sont placés une unité de calcul (10), un transducteur piézoélectrique (8) et un condensateur de mesure (23).

4. Dispositif de surveillance des fonctions vitales du patient selon la revendication 3, **caractérisé en ce que** la deuxième électrode (2) ou la première électrode (25) est fixée à trois endroits autour de la circonférence de la surface touchant la carte de circuits imprimés (7) de manière que chaque fixation permet la déformation du transducteur piézoélectrique (8).

5. Dispositif de surveillance des fonctions vitales du patient selon l'une quelconque des revendications précédentes, **caractérisé en ce qu'**il comprend en outre un condensateur de comparaison (9) qui est connecté électriquement à l'unité de calcul (10).

6. Dispositif de surveillance des fonctions vitales du patient selon la revendication 5, **caractérisé en ce que** le condensateur de comparaison (9) comprend une première électrode de comparaison (26) et une seconde électrode de comparaison (5), la première électrode de comparaison (26) et la seconde électrode de comparaison (5) étant électriquement connectées à l'unité de calcul (10).

7. Dispositif de surveillance des fonctions vitales du patient selon l'une quelconque des revendications précédentes, **caractérisé en ce qu'**il comprend en outre un condensateur d'échantillonnage (11) connecté électriquement à l'unité de calcul (10).

8. Dispositif de surveillance des fonctions vitales du patient selon la revendication 6, **caractérisé en ce qu'**il comprend en outre une électrode de blindage (6) du condensateur de mesure (23), connectée électriquement à l'unité de calcul (10) et ayant le même potentiel électrique que l'électrode de mesure (4), et/ou une électrode de blindage (27) du condensateur de comparaison (9), connectée électriquement à l'unité de calcul (10) qui a le même potentiel électrique que la première électrode de comparaison (26) ou que la seconde électrode de comparaison (5).

9. Dispositif de surveillance des fonctions vitales du patient selon la revendication 8, **caractérisé en ce que** n'importe quel élément du groupe de l'électrode de blindage (6) du condensateur de mesure (23), de l'électrode de blindage (27) du condensateur de comparaison (9), du condensateur de comparaison (9) et du condensateur d'échantillonnage (11) est placé sur la carte de circuits imprimés (7).

10. Dispositif de surveillance des fonctions vitales du patient selon l'une quelconque des revendications 8 à 9, **caractérisé en ce que** l'électrode de blindage (6) du condensateur de mesure (23) est située en face de l'électrode de mesure (4) et l'électrode de blindage (27) du condensateur de comparaison (27) est située en face de la première électrode de comparaison (26) ou de la seconde électrode de comparaison (5).
